(19) Europäisches Patentamt

European Patent Office

Office européen des brevets

(11) **EP 1 156 028 A1**

(12) **EUROPEAN PATENT APPLICATION**

(43) Date of publication:
21.11.2001 Bulletin 2001/47

(51) Int Cl.7: **C07C 68/00**, C07C 69/96

(21) Application number: 00304156.3

(22) Date of filing: 17.05.2000

(84) Designated Contracting States:
**AT BE CH CY DE DK ES FI FR GB GR IE IT LI LU MC NL PT SE**
Designated Extension States:
**AL LT LV MK RO SI**

(71) Applicant: **GENERAL ELECTRIC COMPANY**
**Schenectady, NY 12345 (US)**

(72) Inventors:
• **Shimoda, Tomoaki**
**Ichihara City, 299-0125 Chiba Prefecture (JP)**

• Uno, Kazutoyo
**Chiba City 263-0024, Chiba Prefecture (JP)**
• Tanaka, Masahide
**Ichihara City 299-0114, Chiba Prefecture (JP)**

(74) Representative: **Szary, Anne Catherine, Dr. et al**
**GE London Patent Operation,**
**Essex House,**
**12-13 Essex Street**
**London WC2R 3AA (GB)**

(54) **Method for manufacturing dialkyl carbonate**

(57) The present invention provides a method for efficiently manufacturing a dialkyl carbonate from CO, $O_2$, and an alcohol wherein a cuprous halide and a halogenated hydroacid are used as catalysts. The halogenated hydroacid is used in an amount of 0.001 to 10 mol per mole of the cuprous halide.

EP 1 156 028 A1

**Description**

The present invention relates to a method for manufacturing a dialkyl carbonate. More particularly, it relates to a method for efficiently manufacturing a dialkyl carbonate from CO, $O_2$, and an alcohol.

Aromatic polycarbonates have been widely used in a great many fields in recent years as engineering plastics that have excellent mechanical properties such as impact resistance, and that have excellent heat resistance, transparency, and so on.

The so-called phosgene process, in which an aromatic hydroxy compound such as bisphenol A is reacted with phosgene by interfacial polycondensation, has been used industrially as a method for manufacturing these aromatic polycarbonates. Nevertheless, the phosgene processes currently being used in industrial settings are fraught with problems, such as requiring the use of extremely toxic phosgene, the problem of disposing of the large amounts of by-product sodium chloride, and the problems posed to health and the atmosphere by the methylene chloride that is commonly used as a reaction solvent.

A known method for manufacturing an aromatic polycarbonate besides a phosgene process is a method in which an aromatic dihydroxy compound and a carbonic diester are subjected to transesterification using an alkali metal compound such as sodium hydroxide as a catalyst (melt method). An advantage of this method is that an aromatic polycarbonate can be manufactured inexpensively, and since this method does not require the use of phosgene, methylene chloride, or other such toxic substances, it has been attracting notice in recent years as being preferable from the standpoints of health and environmental safety.

When a polycarbonate is manufactured by this melt method, it is manufactured from carbon monoxide, oxygen, and an alcohol, using a catalyst composed of a cuprous halide such as cuprous chloride. For instance, when methanol is used as the alcohol, dimethyl carbonate is manufactured by the following reaction:

$$2CH_3OH + CO + 1/2O_2 \rightarrow (CH_3O)_2CO + H_2O$$

It is surmised that the cuprous chloride used here as the catalyst forms cuprous methoxychloride in a primary reaction of:

$$2CuCl + 2CH_3OH + 1/2O_2 \rightarrow 2Cu(OCH_3)Cl + H_2O,$$

and is regenerated by a secondary reaction of:

$$2Cu(OCH_3)Cl + CO \rightarrow (CH_3O)CO + 2CuCl.$$

Incidentally, the addition of a halogenated hydroacid to the reaction system in order to enhance the catalytic activity of the cuprous halide used as this catalyst has been disclosed (see Japanese Laid-Open Patent Application 5-194327).

With this method, however, the yield is not always satisfactory, and furthermore the catalyst may clog the reaction tank or pipes, so a problem was that the manufacturing efficiency left something to be desired.

As a result of diligent investigation into a method for manufacturing a dialkyl carbonate at improved manufacturing efficiency conducted in light of this situation, the inventors perfected the present invention upon discovering that when a specific amount of a halogenated hydroacid is used together with a cuprous halide as catalysts, the reaction proceeds in a state in which a high catalytic activity is maintained, and a carbonic diester is obtained at a high yield without any clogging of the reaction tank, pipes, or the like by the catalyst.

The present invention was conceived in light of the above prior art, and provides a method for efficiently manufacturing a dialkyl carbonate from CO, $O_2$, and an alcohol.

The method for manufacturing a dialkyl carbonate pertaining to the present invention is characterized in that, in the manufacture of a dialkyl carbonate using CO, $O_2$, and an alcohol as the starting raw materials, a cuprous halide and a halogenated hydroacid are used as catalysts, and the halogenated hydroacid is used in an amount of 0.001 to 10 mol per mole of the cuprous halide.

It is preferable for the amount in which the cuprous halide is used to be 0.001 to 1.0 mol per mole of methanol.

It is preferable for this cuprous halide to be cuprous chloride.

It is also preferable for the halogenated hydroacid to be hydrochloric acid.

Methanol can be used favorably as the alcohol.

The method for manufacturing a dialkyl carbonate pertaining to the present invention will now be described in specific terms.

The present invention is characterized in that a cuprous halide and a halogenated hydroacid are used as catalysts in the manufacture of a dialkyl carbonate using CO, $O_2$, and an alcohol as the starting raw materials.

There are no particular restrictions on the alcohol used as a starting raw material, but examples include methanol, ethanol, propanol, butanol, isopropanol, isobutanol, and hexanol. Of these, the use of methanol is preferred.

Examples of cuprous halides are cuprous chloride, cuprous fluoride, cuprous bromide, and cuprous iodide. Of these, the use of cuprous chloride is preferred.

Examples of halogenated hydroacids are hydrofluoric acid, hydrochloric acid, hydrobromic acid, and hydroiodic acid, with hydrochloric acid being particularly favorable.

In one embodiment of the method for manufacturing a dialkyl carbonate according to the present invention, the first step is to disperse the cuprous halide used as a catalyst in the above-mentioned alcohol. The amount in which the cuprous halide is used should preferably be between 0.001 and 1.0 mol per mole of methanol.

Next, gases of carbon monoxide and oxygen are introduced under pressure into the cuprous halide/alcohol dispersion. The carbon monoxide and oxygen may be supplied separately to the dispersion, or they may be premixed and then supplied. A gas that does not generate a reaction product in the reaction system, namely, hydrogen, nitrogen, carbon dioxide, methane, argon, or another such inert gas may be present at this time.

The amount in which the carbon monoxide is introduced is preferably greater than the stoichiometric number. Therefore, the molar ratio in which the carbon monoxide and oxygen are introduced (carbon monoxide/oxygen) should preferably be between 3/1 and 100/1, and more preferably between 20/1 and 100/1.

The halogenated hydroacid may be added after being vaporized, or it may be added as an aqueous solution or an alcohol solution. This halogenated hydroacid is used in an amount of 0.001 to 10 mol, and preferably 0.005 to 1.0 mol, per mole of the cuprous halide. Adding the halogenated hydroacid in this amount allows the activity of the cuprous halide used as a catalyst to be raised, and allows the yield of the dialkyl carbonate thus obtained to be raised as well.

The reaction is usually conducted at a temperature of 50 to 200°C, and preferably 100 to 150°C, and under a pressure between atmospheric pressure and 150 atm, and preferably 10 to 100 atm.

A reaction such as this will produce a dialkyl carbonate.

The present invention allows dialkyl carbonate to be converted from alcohol at a rate of 7 to 30%. Accordingly, the yield at which the dialkyl carbonate is obtained can be raised.

The produced dialkyl carbonate can be recovered by utilizing a known separation method, such as distillation, filtration, decanting, centrifugation, demixing, or osmotic membrane separation. Two or more types of these separation methods may also be combined.

The cuprous halide, halogenated hydroacid, unreacted alcohol, and the like contained in the reaction solution after the recovery of the produced dialkyl carbonate can be recovered and reused.

This reaction can be carried out using a batch reaction tank, or using a continuous reaction tank. The use of an autoclave or other such pressure resistant vessel is preferable.

When a continuous reaction tank is used, the alcohol, halogenated hydroacid, carbon monoxide, and oxygen are allowed to react under the above-mentioned conditions by being supplied to a solution containing an alcohol, water, and a cuprous halide, then the reaction solution containing the produced dialkyl carbonate, water, and alcohol, and the unreacted carbon monoxide and water vapor are taken out, the dialkyl carbonate and the water are removed from the reaction solution, and the other components are recirculated back to the reaction system.

It is acceptable for unrecovered dialkyl carbonate to be contained in the reaction solution to which the alcohol, halogenated hydroacid, carbon monoxide, and oxygen are supplied. It is preferable for the reaction solution to which the alcohol, halogenated hydroacid, carbon monoxide, and oxygen are supplied to have an alcohol concentration of 30 to 80 wt%, and even more preferably 35 to 80%, and to have a water concentration of 1 to 10 wt%, and even more preferably 2 to 7 wt%.

With the method for manufacturing a dialkyl carbonate pertaining to the present invention, a dialkyl carbonate can be manufactured efficiently, in a state of high catalytic activity, and without any clogging of the pipes or reaction tank by the catalyst. Also, the use of a dialkyl carbonate obtained in this manner in the manufacture of a polycarbonate makes it possible to obtain a polycarbonate with improved coloring, and this polycarbonate can be used favorably for ordinary molding materials, in sheeting and other such construction materials, in automotive headlamp lenses, eyeglasses, and other such optical lenses, in optical recording materials, and so on, and will be particularly favorable as an optical disk molding material.

Working Examples

The present invention will now be described in further detail through working examples, but the present invention is not limited to or by these working examples.

The physical properties given in the working examples of the present invention were measured as follows.

Quantification of Solids

The reaction solution containing solids in the autoclave after the reaction was filtered, and the filtered solids were quantified, after which the solids amount was divided by the reaction solution weight, and this quotient was used as an index of clogging.
The greater is the amount of solids, the more likely clogging is to happen.

Working Example 1

80 g of methanol and 20 g of cuprous chloride were put in an autoclave with a volume of 300 mL and sealed inside.
The temperature in the autoclave was then raised to 125°C, carbon monoxide gas and oxygen gas were supplied to the autoclave such that the total pressure would be 25 kg/cm$^2$ G and the oxygen concentration would be 5% or less, a 36% hydrochloric acid aqueous solution was further supplied such that the molar ratio between cuprous chloride and hydrogen chloride (HCl/CuCl) would be 0.27, and the components were allowed to react for 4 hours at 125°C.
After the autoclave was cooled, the unreacted gas was slowly purged, and the reaction solution was taken out and subjected to quantitative analysis by gas chromatography, which revealed that the methanol conversion rate was 9.9 mol%, the amount of dimethyl carbonate produced was 21.8 g, and the yield was 9.7 mol%. The solids content was 4.3 wt% with respect to the reaction solution.
By-products were confirmed to be methylal, methyl chloride, and dimethyl ether.

Working Examples 2 to 4

Dimethyl carbonate was manufactured in the same manner as in Working Example 1 except that the amount of hydrochloric acid used in Working Example 1 was changed as shown in Table 1. The yield of dimethyl carbonate thus obtained and the solids content in the reaction [solution] are also shown in Table 1.

Comparative Example 1

Dimethyl carbonate was manufactured in the same manner as in Working Example 1 except that the hydrochloric acid used in Working Example 1 was not used. The solids content here was 12.2 wt%.
Table 1 gives the yield of the obtained dimethyl carbonate and the solids content in the reaction.

Table 1

| | HCl/CuCl molar ratio | $CH_3OCO_2CH_3$ mol % | Solids weight/total amount of Reaction solution (wt%) |
|---|---|---|---|
| Working Example 1 | 0.27 | 9.7 | 4.3 |
| Working Example 2 | 0.33 | 10.9 | 3.9 |
| Working Example 3 | 0.41 | 12.2 | 3.7 |
| Working Example 4 | 0.80 | 20.5 | 2.0 |
| Comparative Example 1 | 0.00 | 7.5 | 12.2 |

**Claims**

1.   A method for manufacturing a dialkyl carbonate using carbon monoxide, oxygen, and an alcohol as the starting raw materials,

wherein in said method for manufacturing a dialkyl carbonate a cuprous halide and a halogenated hydroacid are used as catalysts, and

the halogenated hydroacid is used in an amount of 0.001 to 10 mol per mole of the cuprous halide.

2. The method for manufacturing a dialkyl carbonate according to claim 1, wherein the amount of cuprous halide used is 0.001 to 1.0 mol per mole of alcohol.

3. The method for manufacturing a dialkyl carbonate according to claim 1 or claim 2, wherein the cuprous halide is cuprous chloride.

4. The method for manufacturing a dialkyl carbonate according to any preceding claim, wherein the halogenated hydroacid is hydrochloric acid.

5. The method for manufacturing a dialkyl carbonate according to any preceding claim, wherein the alcohol is methanol.

**European Patent Office**

## EUROPEAN SEARCH REPORT

Application Number

EP 00 30 4156

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 686 644 A (FRANCO RIVETTI) 11 November 1997 (1997-11-11) * column 4 - column 6; examples 2-4 * * column 6 - column 8; claims * ----- | 1-5 | C07C68/00 C07C69/96 |

**TECHNICAL FIELDS SEARCHED (Int.Cl.7)**

C07C

The present search report has been drawn up for all claims

| Place of search | Date of completion of the search | Examiner |
|---|---|---|
| THE HAGUE | 12 October 2000 | Kinzinger, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 1 156 028 A1

## ANNEX TO THE EUROPEAN SEARCH REPORT
## ON EUROPEAN PATENT APPLICATION NO.

EP 00 30 4156

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2000

| Patent document cited in search report | | Publication date | Patent family member(s) | | Publication date |
|---|---|---|---|---|---|
| US 5686644 | A | 11-11-1997 | IT | 1251853 B | 26-05-1995 |
| | | | AT | 160768 T | 15-12-1997 |
| | | | AU | 656805 B | 16-02-1995 |
| | | | AU | 2526392 A | 25-03-1993 |
| | | | DE | 69223367 D | 15-01-1998 |
| | | | DE | 69223367 T | 14-05-1998 |
| | | | DK | 534545 T | 10-08-1998 |
| | | | EP | 0534545 A | 31-03-1993 |
| | | | ES | 2109976 T | 01-02-1998 |
| | | | GR | 3025894 T | 30-04-1998 |
| | | | JP | 5194327 A | 03-08-1993 |
| | | | KR | 9606550 B | 17-05-1996 |
| | | | MX | 9205363 A | 01-05-1993 |
| | | | RU | 2056404 C | 20-03-1996 |
| | | | ZA | 9207212 A | 24-03-1993 |

EPO FORM P0459

For more details about this annex : see Official Journal of the European Patent Office, No. 12/82

7